# EUROPEAN PATENT SPECIFICATION

(11) **EP 3 934 755 B1**
(45) Date of publication and mention of the grant of the patent: **27.03.2024**
(21) Application number: 20709307.1
(22) Date of filing: 03.03.2020
(51) Int. Cl.: A61P 31/04, A61K 31/352, A61K 31/496, A61K 31/7036, A61K 38/14, A61K 45/06, A61K 31/7048, A61K 31/05, A61K 31/353, A61K 38/12

(54) **USE OF CANNABIDIOL IN COMBINATION WITH ANTIBIOTICS**
VERWENDUNG VON CANNABIDIOL IN KOMBINATION MIT ANTIBIOTIKA
UTILISATION DE CANNABIDIOL EN COMBINAISON AVEC DES ANTIBIOTIQUES

(30) Priority: 06.03.2019 GB 201903002
(43) Date of publication of application: 12.01.2022
(73) Proprietor: GW Research Limited, Cambridge, Cambridgeshire CB24 9BZ (GB)
(72) Inventor: GUY, Geoffrey, Cambridge Cambridgeshire CB24 9BZ (GB); KNAPPERTZ, Volker, Cambridge Cambridgeshire CB24 9BZ (GB)
(74) Representative: HGF
(86) International application number: PCT/GB2020/050508
(87) International publication number: WO 2020/178576

(56) References cited:
- WO-A1-2018/011813
- WO-A1-2018/148786
- WO-A1-2018/234301
- PRALAY SANKAR CHAKARBORTY ET AL: "Synergistic interaction of Cannabis and Garlic with commercial antibiotics", INTERNATIONAL JOURNAL OF PHARMACOGNOSY AND PHYTOCHEMICAL RESEARCH, vol. 7, no. 1, 1 January 2015 (2015-01-01) , pages 193-196, XP055459377,
- UCHINI S. KOSGODAGE ET AL: "Cannabidiol Is a Novel Modulator of Bacterial Membrane Vesicles", FRONTIERS IN CELLULAR AND INFECTION MICROBIOLOGY, vol. 9, 10 September 2019 (2019-09-10), XP055690557, DOI: 10.3389/fcimb.2019.00324

## Description

The present invention relates to a combination of cannabidiol (CBD), a phytocannabinoid which is naturally produced by Cannabis sativa plants or can be made synthetically, with an antibiotic for use in the treatment of bacterial infection caused by Gram-negative bacteria, wherein the antibiotic is rifampicin or erythromycin.

Preferably the CBD increases the bactericidal action of antibiotics. The bactericidal action is used against Gram-negative bacteria.

In a further disclosure, which is not claimed, the CBD increases the antibiotic effects of Kanamycin in Gram-positive bacteria.

Such combinations may help overcome antibiotic resistance.

Preferably the CBD used is in the form of a highly purified extract of cannabis such that the CBD is present at greater than 95% of the total extract (w/w) and the other components of the extract are characterised. In particular tetrahydrocannabinol (THC) has been substantially removed to a level of not more than 0.1% (w/w). Alternatively, it is a synthetically produced CBD.

In use the CBD is used concomitantly with one or more antibiotics. Alternatively, the CBD may be formulated for administration separately, sequentially or simultaneously with one or more antibiotics or the combination may be provided in a single dosage form. Where the CBD is formulated for administration separately, sequentially or simultaneously it may be provided as a kit or together with instructions to administer the one or more components in the manner indicated.

### BACKGROUND TO THE INVENTION

Outer membrane vesicles (OMVs), also called membrane vesicles (MVs), are released from Gram-positive and Gram-negative bacteria and participate in inter-bacterial communication, including via transfer of cargo molecules (Jan, 2017; Toyofuku et al., 2019).

OMVs are released in more abundance from Gram-negative, than Gram-positive bacteria and OMV production seems crucial for bacterial survival (Jan, 2017). OMVs play for example roles in biofilm formation and can also disseminate toxins in the host (Wang et al., 2015).

OMVs also participate in host-pathogen interactions (Toyofuku et al., 2019), and may also play important roles in antibiotic resistance; including by protecting biofilms from antibiotics (Manning and Kuehn, 2011). Furthermore, OMVs from Porphyromonas gingivalis have for example been linked to metabolic remodelling in the host (Fleetwood et al., 2017), while OMVs from Neisseria gonorrhoeae have been shown to target host mitochondria and to induce macrophage death (Deo et al., 2018).

The regulation of bacterial OMV release may therefore be of great importance, both in relation to inter-bacterial communication, including biofilm formation, their host interactions as commensals, as well as in host-pathogen interactions and antibiotic resistance.

Cannabidiol (CBD) is a phytocannabinoid commonly found in Cannabis sativa and linked to various anti- inflammatory (Martin-Moreno et al., 2011), anti-cancerous (Pisanti et al., 2017; Kosgodage et al., 2018) as well as anti-bacterial functions (Hernandez-Cervantes et al., 2017). WO 2018/234301 A1 discloses cannabidiol as a potential helper compound in the treatment of bacterial infections.
While immunoregulatory roles for cannabinoids have been reported in infectious disease (reviewed in Hernandez-Cervantes et al., 2017), and C. sativa has been identified as a natural compound with a capability of controlling bacterial infections, including a strong anti-bacterial activity against antibiotic resistant strains (Appendino et al., 2008), a link between CBD and OMV release has hitherto not been investigated in bacteria.

Novel strategies to tackle antibiotic resistance are urgently needed. Therefore, combinatory application of effective OMV-inhibitors and selected antibiotics may offer tailored treatment for infectious disease caused by antibiotic resistant bacteria.

The present invention provides data to demonstrate the combined effect of CBD with certain antibiotics. These data provide evidence that the combination is of particular benefit in increasing the anti-bacterial efficacy in Gram-negative bacteria.

### BRIEF SUMMARY OF THE DISCLOSURE

The present invention provides a combination of cannabidiol (CBD) with an antibiotic for use in the treatment of bacterial infection caused b a Gram-negative bacterium, wherein the antibiotic is rifampicin or erythromycin.

The bacterial infection is caused by a Gram-negative bacterium. In a disclosure, which is not part of the claimed invention, the bacterial infection is caused by a Gram-positive bacterium.

The antibiotic is taken from the group consisting of: Colistin; Rifampicin; Erythromycin; Kanamycin and Vancomycin. Only compositions wherein the antibiotic is rifampicin or erythromycin are according to the claimed invention.

Preferably the CBD is present as a highly purified extract of cannabis which comprises at least 95% (w/w) CBD. More preferably the extract comprises at least 96% (w/w) CBD, more preferably the extract comprises at least 97% (w/w) CBD, more preferably still the extract comprises at least 98% (w/w) CBD.

Preferably the extract comprises up to 0.1% (w/w) THC. More preferably the THC is present at a concentration of between 0.02 and 0.1% (w/w).

In an alternative embodiment the CBD is present as a synthetic compound.

Preferably the dose of CBD is between 0.1 and 1000 mg/kg/day.

Preferably the patient is a human.

### BRIEF DESCRIPTION OF THE DRAWINGS

Embodiments of the invention are further described hereinafter with reference to the accompanying drawings, in which:

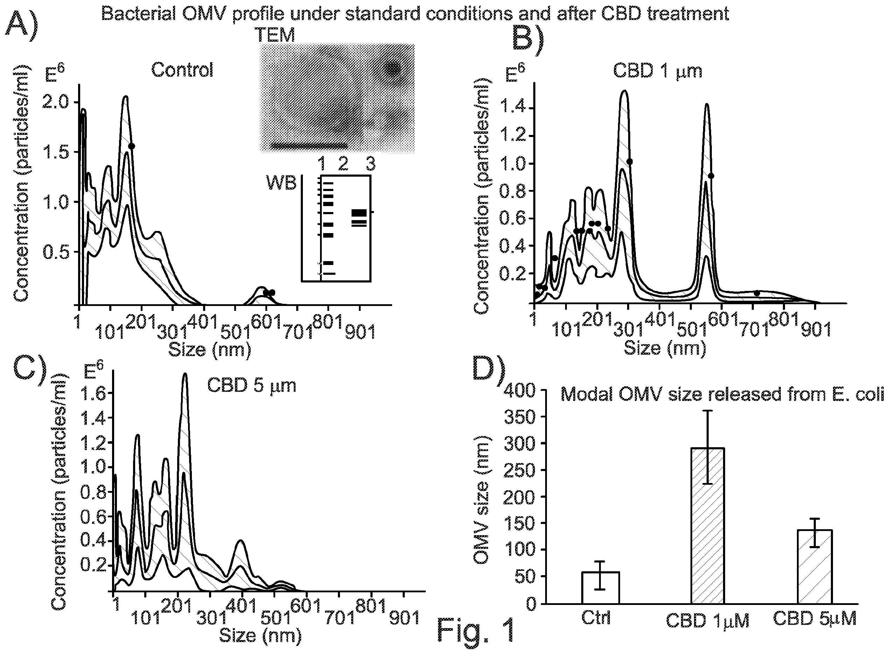

Figure 1 describes bacterial OMV profile under standard conditions and after CBD treatment. A) OMVs released from untreated *E. coli* shown by NTA analysis (Nanosight); Transmission electron microscopy (TEM, scale bar = 200 nm) and Western blotting with the OMV-specific marker OmpC. B) NTA analysis showing OMV release from *E. coli* after 1h CBD treatment (1 µM); C) NTA analysis showing OMV release from *E. coli* after 1h CBD treatment (5 µM). D) Modal size of OMVs released from *E. coli* under normal culture conditions compared to CBD treatment. Error bars indicate SEM;

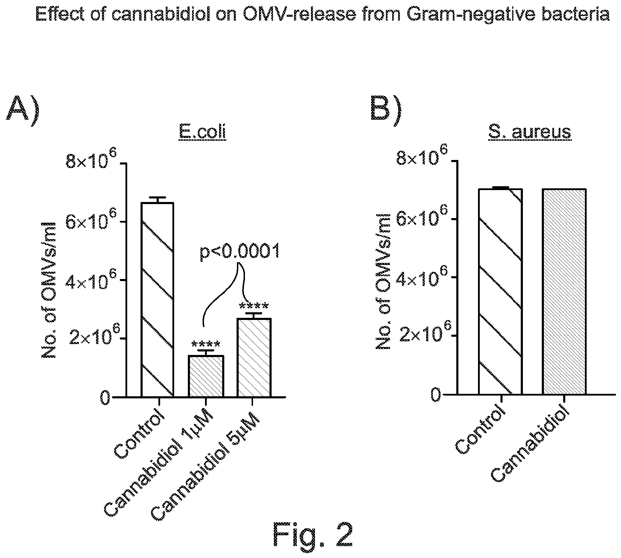

Figure 2 describes the effect of cannabidiol on OMV-release from Gram-negative bacteria. A) OMV release from *E. coli* was significantly reduced after CBD treatment, with lower dose of CBD being more effective. B) Membrane vesicle release from *S*. *aureus* was not significantly affected by CBD treatment;

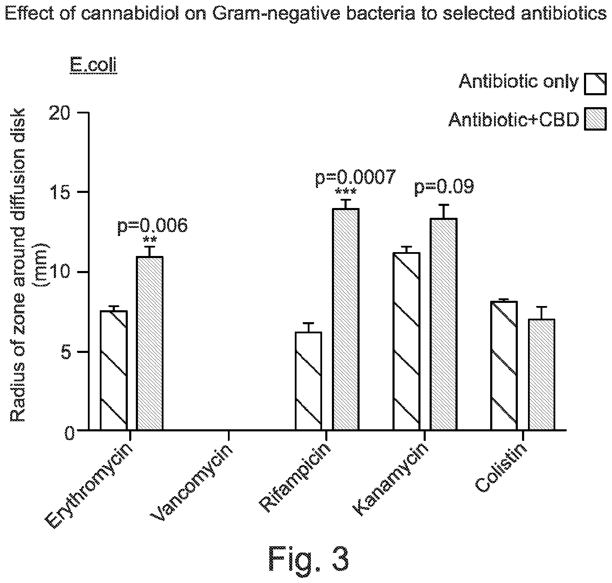

Figure 3 describes the effect of cannabidiol on Gram-negative bacteria to selected antibiotics. Combinatory treatment of CBD with a range of antibiotics showed enhanced CBD-mediated antibacterial effects on *E. coli,* as assessed by increased radius of zone around the diffusion disk. CBD was most effective in combination with Rifampicin and Erythromycin. Exact p-values are shown;

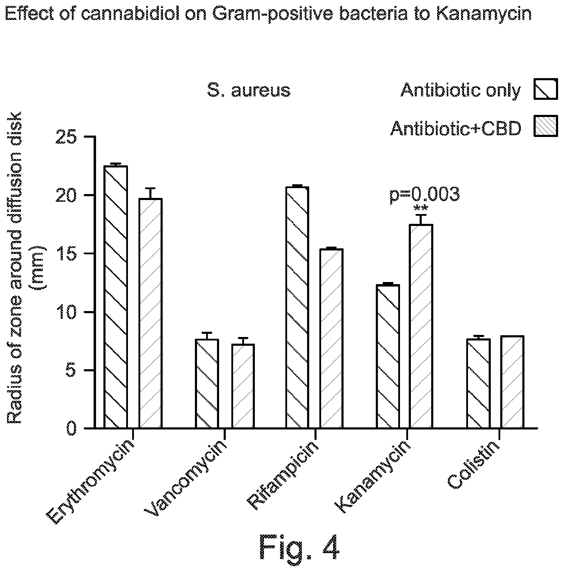

Figure 4 describes the effect of cannabidiol on Gram-positive bacteria to Kanamycin. Combinatory treatment of CBD with a range of antibiotics showed enhanced antibacterial effects of Kanamycin only on *S*. *aureus,* as assessed by increased radius of zone around the diffusion disk. CBD did not enhance anti-bacterial activity against Gram-positive bacteria for the other antibiotics tested and reduced antibacterial effects of both Erythromycin and Rifampicin. Exact p-values are shown; and

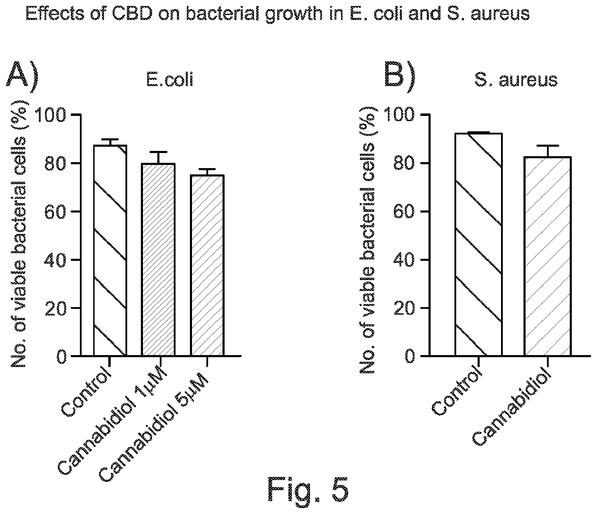

Figure 5 shows the effects of CBD on bacterial growth in *E. coli* and *S*. *aureus,* exact p-values are shown.

### DEFINITIONS

Definitions of some of the terms used to describe the invention are detailed in Table 1 below:

The cannabinoids described in the present application are listed below along with their standard abbreviations.

**Table 1. Cannabinoids and their abbreviations**

| | | |
|---|---|---|
| CBD | Cannabidiol | 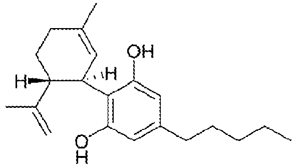 |
| THC | Tetrahydrocannabinol | 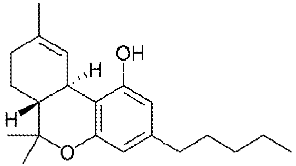 |

The table above is not exhaustive and merely details the cannabinoids which are identified in the present application for reference. So far over 100 different cannabinoids have been identified and these cannabinoids can be split into different groups as follows: Phytocannabinoids; Endocannabinoids and Synthetic cannabinoids (which may be novel cannabinoids or synthetically produced phytocannabinoids or endocannabinoids). Such cannabinoids are disclosed in Handbook of Cannabis, Roger Pertwee, Chapter 1, pages 3 to 15.

"Phytocannabinoids" are cannabinoids that originate from nature and can be found in the cannabis plant. The phytocannabinoids can be isolated from plants to produce a highly purified extract or can be reproduced synthetically.

"Botanical drug substance" or "BDS" is defined in the Guidance for Industry Botanical Drug Products Draft Guidance, August 2000, US Department of Health and Human Services, Food and Drug Administration Centre for Drug Evaluation and Research as: "A drug substance derived from one or more plants, algae, or macroscopic fungi. It is prepared from botanical raw materials by one or more of the following processes: pulverisation, decoction, expression, aqueous extraction, ethanolic extraction, or other similar processes. A botanical drug substance does not include a highly purified or chemically modified substance derived from natural sources. Thus, in the case of cannabis, "botanical drug substances" derived from cannabis plants do not include highly purified, Pharmacopoeial grade cannabinoids.

"Highly purified cannabinoids" are defined as cannabinoids that have been extracted from the cannabis plant and purified to the extent that other cannabinoids and non-cannabinoid components that are co-extracted with the cannabinoids have been removed, such that the highly purified cannabinoid is greater than or equal to 95% (w/w) pure.

"Synthetic cannabinoids" are compounds that have a cannabinoid or cannabinoid-like structure and are manufactured using chemical means rather than by the plant.

Phytocannabinoids can be obtained as either the neutral (decarboxylated form) or the carboxylic acid form depending on the method used to extract the cannabinoids. For example, it is known that heating the carboxylic acid form will cause most of the carboxylic acid form to decarboxylate into the neutral form.

### DETAILED DESCRIPTION

The following example demonstrates that CBD when administered alone was able to effectively inhibit outer membrane vesicles (OMVs) release from an example of Gram-negative bacteria, *E. coli.* The CBD did not have significant effects on membrane vesicle release in an example of Gram-positive bacteria *S*. *aureus.*

When applied in combination with a range of antibiotics, the CBD increased anti-bacterial effects of selected antibiotics, depending on bacteria type.

### EXAMPLE 1: MODULATION OF BACTERIAL OUTER MEMBRANE VESICLES AND SENSITISATION OF BACTERIA TO ANTIBIOTICS BY CANNABIDIOL

### Materials and Methods

### OMV isolation

*E. coli* and *S*. *aureus* cultures were maintained by plating on Müeller-Hinton agar plates and weekly sub-culturing was performed according to previously established methods (Iqbal et.al. 2013).

Before OMV isolation, all bacterial growth medium was pre-treated before use by ultracentrifugation at 75,000 g for 24 h, to ensure minimum contamination with extracellular vesicles (EVs) from the medium.

For OMV isolation, bacteria were grown in EV-free medium (as described above) for 24 h at 37°C and 5% CO2, the culture medium collected and centrifuged once at 400g for 10 min for removal of cells, followed by centrifugation at 4000g for 1h at 4°C to remove cell debris. The resultant supernatant was then centrifuged for 1h at 25,000g at 4°C and the isolated OMV pellet was resuspended in DPBS (ultracentrifuged and sterile filtered using a 0.22µm filter) and centrifuged again at 25,000g for 1h at 4°C. The resulting OMV pellet was sterile filtered (0.4µm) once, then resuspended in sterile filtered DPBS. OMVs were then either used immediately or stored at -80 °C for further experiments.

### Transmission Electron Microscopy (TEM) imaging of OMVs

A suspension of isolated OMVs (1.4 × 108 OMVs/ml) was used for TEM imaging. OMV samples (10µL) were applied to mesh copper grids, prepared with glow discharged carbon support films, and incubated for 2 min. The grids were then washed five times with 50µl of 1% aqueous uranyl acetate. Grids were left to dry for 5min before being viewed. Micrographs were taken with a JEOL JEM 1230 transmission electron microscope (JEOL, U.S.A.) operated at 80 kV at a magnification of 80,000 to 100,000. Digital images were recorded using a Morada CCD camera (EMSIS, Germany) and processed via iTEM (EMSIS).

### Western blotting

Protein was isolated from OMV pellets using RIPA+ buffer (Sigma, U.K.), pipetting gently and shaking the pellets on ice for 2h, where after samples were centrifuged at 16,000g at 4°C for 20 min and the resulting supernatant collected for protein analysis. Samples were prepared in 2x Lammli buffer, boiled at 95°C for 5min, electrophoresed by SDS-PAGE on 5-20% TGX gels (BioRad, U.K.), followed by semi-dry Western blotting.

Approximately 10µg of protein was loaded per lane and even protein transfer was assessed by Ponceau S staining (Sigma, U.K.). Blocking of membranes was performed for 1h at room temperature in 5% BSA in TBS-T. The membranes were then incubated in anti-OmpC (Outer-membrane protein C antibody; orb6940, Biorbyt, U.K.) overnight at 4°C, followed by washing in TBS-T and incubation for 1h in anti-rabbit-HRP conjugated secondary antibody at RT. Visualisation was performed using ECL (Amersham, U.K.) and the UVP BioDoc-ITTM System (U.K.).

### Nanoparticle tracking analysis

OMVs were isolated from control and CBD-treated bacterial cultures as described above. Nanoparticle tracking analysis (NTA) was performed using the Nanosight LM10 (Malvern, U.K.), equipped with a 405 nm diode laser and a sCMOS camera.

Samples were diluted 1:50 in sterile-filtered EV-free DPBS and the number of particles in the field of view was maintained in the range of 20-40 with a minimum concentration of samples at 5 × 107 particles/ml. Camera settings were according to the manufacturer's instructions (Malvern), five 30 sec videos per sample were recorded and replicate histograms averaged. Each experiment was repeated three times.

### CBD-mediated OMV inhibition in E. coli and S. aureus

*E. coli* and *S*. *aureus* cultures were prepared as before. CBD (1 or 5 µM) were added in triplicates and incubated for 1h at 37°C in 5% CO₂.

The experiment was repeated three times and the OMVs were quantified using NTA analysis. Cell viability was assessed after 1h treatment with CBD compared to controls and before the start of every experiment using viable count.

### Disc Diffusion Test

Discs were impregnated with the following antibodies: Colistin (10 µg/ml), Rifampicin (15 µg/ml), Erythromycin (15 µg/ml), Kanamycin (30 µg/ml) and Vancomycin (5 µg/ml).

Concentration of the antibiotics used was based on previously published and established MIC values (Moskowitz et al., 2010; Rojas et al., 2017; Goldstein et al., 2018; Maclayton et al., 2006, Kshetry et al., 2016).

*E. coli* and *S*. *aureus* agar plates were prepared for the disc diffusion test by soaking a sterile paper disc in 5µM CBD and placing it in the middle of the agar plate, while the impregnated antibiotic discs were placed equidistant to the CBD disc. Zone of inhibition was assessed after 24h.

### Statistical Analysis

Histograms and graphs were prepared, and statistical analysis was performed using GraphPad Prism version 8 (GraphPad Software, San Diego, U.S.A.). One-way ANOVA and t-test analysis were performed, followed by Tukey's post-hoc analysis. Histograms represent mean of data, with error bars representing standard error of mean (SEM). Significant differences were considered as p ≤ 0.05.

### Results

### Characterisation of OMVs

Isolated OMVs were assessed by morphology using transmission electron microscopy, revealing a poly-dispersed population mainly in the size range of mainly 20 - 230 nm in diameter (Figure 1A).

NTA analysis verified that the majority of the vesicle population fell in a similar size range, with smaller peaks around 400 nm under standard culture conditions (Figure 1A). Furthermore, Western blotting showed positive for the OMV specific marker OmpC (Fig. 1A).

### Inhibitory effects of CBD on OMV release from E. coli and S. aureus

CBD changed the OMV-release profile from *E. coli* compared to control treatment (Fig. 1A-D), as also seen in a shift in modal size of membrane vesicles released (Fig. 1D). CBD had a strongly significant inhibitory effect on total OMV release at both concentrations tested (1 and 5µM respectively) (Fig. 2A).

In addition, the lower dose of CBD (1µM) had stronger OMV-inhibitory effects (73 % reduction, p<0.0001) than 5µM CBD (54 % reduction, p<0.0001) and furthermore resulted in a marked peak at 500 nm (Fig. 1B), which otherwise was negligible in the control and 5µM CBD treated *E. coli.*

Contrary to what was observed for the Gram-negative *E. coli,* CBD treatment had no effect on membrane vesicle release from the Gram-positive bacterium *S*. *aureus* (Fig. 2B). CBD treatment affects antibiotic sensitivity in *E. coli*

CBD treatment, when applied in combination with a range of antibiotics tested, was found to sensitise *E. coli* to selected antibiotics, as assessed by increased radius of zone using the disk diffusion test (Fig. 3).

Significantly enhanced antibacterial effects were found for Erythromycin (35% increase; p=0.006) and Rifampicin (50% increase; p=0.0007), when combined with CBD (5µM) treatment, compared to antibiotic treatment alone. Antibacterial effects of Kanamycin, which is not part of the claimed invention, were also somewhat increased, albeit not significant (18%; p=0.09).

### CBD-mediated effects on antibiotic sensitivity in S. aureus (Reference)

In *S*. *aureus,* CBD increased the antibiotic activity of Kanamycin only (30%; p=0.003), as assessed by increased radius of zone around the diffusion disk. CBD did not enhance anti-bacterial activity for the other antibiotics tested and reduced antibacterial effects of both Erythromycin and Rifampicin.

### Conclusions

The present example demonstrates that CBD significantly reduced OMV-release in *E. coli,* an example of a gram-negative bacterium, but had negligible effects on membrane vesicle release in *S*. *aureus,* an example of a gram-positive bacterium.

Surprisingly, we found that lower doses of CBD had a stronger OMV inhibitory effect in *E. coli* than a higher 5µM dose.

When assessing the effectiveness of CBD to enhance susceptibility of Gram-positive and Gram-negative bacterial species to a range of antibiotics, OMV-inhibition rendered *E. coli* significantly more sensitive to Erythromycin and Rifampicin and somewhat to Kanamycin, but not to Colisitin.

CBD did not increase antibacterial effects of Vancomycin on *E. coli,* confirming its limited effectiveness on Gram-negative species and the previously established resistance of *E*. *coli* to Vancomycin, due to its inability to significantly penetrate the outer membrane (Zhou et al., 2015).

In the gram-positive bacterium *S*. *aureus,* CBD increased bactericidal activity of Kanamycin only. The reduced ability of CBD to sensitise a gram-positive bacterium to antibiotics, compared to the significantly higher effects in a gram-negative bacterium, tallied in with CBD's ability to regulate OMV-release, indicating a significant contribution of OMVs to antibiotic resistance.

This also shows that selective OMV-inhibitors that target membrane vesicles from specific bacteria species, such as CBD here, could be applied in combination with selected antibiotics for tailored antibiotic treatment to tackle antibiotic resistance.

CBD, in combination with specific antibiotics, may thus be used to selectively target bacteria to sensitise them to antibiotic treatment and overcome antibiotic resistance.

## Claims

1. A combination of cannabidiol (CBD) with an antibiotic for use in the treatment of bacterial infection caused by a Gram-negative bacterium, wherein the antibiotic is Rifampicin or Erythromycin.

2. A combination of CBD with an antibiotic for use according to claim 1, wherein the CBD is present as a highly purified extract of cannabis comprising at least 95% (w/w) CBD.

3. A combination of CBD with an antibiotic for use according to claim 2, wherein the extract comprises up to 0.1% (w/w) THC.

4. A combination of CBD with an antibiotic for use according to claim 3, wherein the THC is present at a concentration of between 0.02 and 0.1% (w/w).

5. A combination of CBD with an antibiotic for use according to any of the preceding claims, wherein the dose of CBD is between 0.1 and 1000 mg/kg/day.

## Patentansprüche

1. Kombination von Cannabidiol (CBD) mit einem Antibiotikum zur Verwendung bei der Behandlung einer bakteriellen Infektion, die durch ein gramnegatives Bakterium verursacht wird, wobei das Antibiotikum Rifampicin oder Erythromycin ist.

2. Kombination von CBD mit einem Antibiotikum zur Verwendung nach Anspruch 1, wobei das CBD als ein hochgereinigter Cannabisextrakt vorliegt, der mindestens 95% (Gew./Gew.) CBD umfasst.

3. Kombination von CBD mit einem Antibiotikum zur Verwendung nach Anspruch 2, wobei der Extrakt bis zu 0,1% (Gew./Gew.) THC umfasst.

4. Kombination von CBD mit einem Antibiotikum zur Verwendung nach Anspruch 3, wobei das THC in einer Konzentration zwischen 0,02 und 0,1% (Gew./Gew.) vorliegt.

5. Kombination von CBD mit einem Antibiotikum zur Verwendung nach einem der vorhergehenden Ansprüche, wobei die CBD-Dosis zwischen 0,1 und 1000 mg/kg/Tag liegt.

## Revendications

1. Combinaison de cannabidiol (CBD) et d'un antibiotique pour utilisation dans le traitement d'une infection bactérienne causée par une bactérie à Gram négatif, dans laquelle l'antibiotique est la Rifampicine ou l'Erythromycine.

2. Combinaison de CBD et d'un antibiotique pour utilisation selon la revendication 1, dans laquelle le CBD est présent sous la forme d'un extrait hautement purifié de cannabis comprenant au moins 95 % (p/p) de CBD.

3. Combinaison de CBD et d'un antibiotique pour utilisation selon la revendication 2, dans laquelle l'extrait comprend jusqu'à 0,1 % (p/p) de THC.

4. Combinaison de CBD et d'un antibiotique pour utilisation selon la revendication 3, dans laquelle le THC est présent à une concentration comprise entre 0,02 et 0,1 % (p/p).

5. Combinaison de CBD et d'un antibiotique pour utilisation selon l'une quelconque des revendications précédentes, dans laquelle la dose de CBD est comprise entre 0,1 et 1000 mg/kg/jour.
